Europäisches Patentamt

European Patent Office

Office européen des brevets

(19)

(11) **EP 0 685 201 B1**

(12) **FASCICULE DE BREVET EUROPEEN**

(45) Date de publication et mention
de la délivrance du brevet:
**13.02.2002 Bulletin 2002/07**

(51) Int Cl.⁷: **A61B 6/14**, H04N 5/32,
H04N 3/15, A61B 6/03

(21) Numéro de dépôt: **95401242.3**

(22) Date de dépôt: **29.05.1995**

(54) **Appareil de radiodiagnostic comportant un scintillateur avec un capteur à transfert de charge**

Röntgendiagnostikeinrichtung mit Szintillator und CCD-Sensor

Radiodiagnostic device having a scintillator with a CCD sensor

(84) Etats contractants désignés:
**DE ES FR GB IT**

(30) Priorité: **31.05.1994 FR 9406628**

(43) Date de publication de la demande:
**06.12.1995 Bulletin 1995/49**

(73) Titulaire: **TROPHY RADIOLOGIE**
**F-94300 Vincennes (FR)**

(72) Inventeurs:
• **Augais, Thierry**
**F-91190 Gif-Sur-Yvette (FR)**
• **Thorez, Manuel**
**F-94360 Brie-Sur-Marne (FR)**

(74) Mandataire: **Berger, Helmut et al**
**Cabinet WEINSTEIN 56 A, rue du Faubourg**
**Saint-Honoré**
**75008 Paris (FR)**

(56) Documents cités:
**EP-A- 0 138 625**     **EP-A- 0 357 944**
**EP-A- 0 366 235**     **EP-A- 0 369 585**
**WO-A-90/14793**     **DE-A- 3 937 077**
**US-A- 4 628 356**

## Description

L'invention concerne un appareil de radiodiagnostic pour établir des prises de vue panoramiques et avantageusement tomographiques d'un objet tel que de la mâchoire d'un patient, du type décrit dans le préambule de la revendication 1.

Un appareil de ce type est décrit dans le document WO-A-9014793. Dans l'appareil décrit dans ce document, la source de rayon et le détecteur des rayons ayant traversé l'objet à radiographier sont déplacés tous les deux pour prendre des images panoramiques. Le détecteur est stationnaire par rapport à la source des rayons X. L'objectif à atteindre dans ce document est d'obtenir une image de rayons X sans distorsion, d'un objet qui a une courbure irrégulière. A cette fin, il est préconisé dans ce document de simuler le mouvement de film en faisant varier le temps d'intégration pour les colonnes successives des signaux d'éléments d'image.

Les appareils de radiodiagnostic de ce type présentent l'inconvénient qu'ils sont incompatibles avec les appareils de radiodiagnostic classiques, qui utilisent des films radiographiques à déplacement linéaire ou rotatif, inclus dans une cassette ou un tambour porte-film. Cette incompatibilité est due à la réalisation des moyens de commande du décalage des lignes de charges.

Par conséquent un médecin qui souhaite disposer d'un appareil apportant les avantages que procure un capteur du type à couplage de charges est obligé de remplacer son appareil classique à film radiographique par un nouvel appareil spécifique équipé du capteur à couplage de charges.

L'invention a pour but d'éviter cet inconvénient et de proposer un appareil mettant en oeuvre un capteur du type DTC (Dispositif à Transfert de Charges ou CCD, selon la terminologie anglo-saxone), qui est compatible avec les appareils classiques.

Pour atteindre ce but, l'appareil selon l'invention comporte les caractéristiques qui sont énoncées dans la partie caractérisante de la revendication 1.

Par conséquent, la particularité de l'invention réside dans le fait que l'appareil proposé comporte une partie mobile se déplaçant derrière la fente secondaire à la vitesse à laquelle se déplaceraient un film radiographique et un détecteur de cette vitesse ainsi qu'une unité de conversion de la vitesse en une fréquence équivalente de décalage des lignes du capteur à transfert de charge.

Différemment de l'invention, dans le document D1, le mouvement de film est seulement simulé en faisant varier le temps d'intégration pour les colonnes successives des signaux d'éléments d'image. Par conséquent, l'invention ne découle pas de façon évidente de ce document.

Le document EP-A-0 366 235 n'apporte également aucun enseignement qui permettrait la réalisation de l'invention. Ce document concerne un système de commande de la vitesse du déplacement d'une bande, qui utilise certes une matrice CCD, mais dans un contexte qui n'a rien à voir avec l'invention.

D'autres caractéristiques avantageuses de l'invention sont énoncées dans des revendications dépendantes.

L'invention sera mieux comprise et d'autres buts, caractéristiques, détails et avantages de celle-ci apparaîtront plus clairement dans la description explicative qui va suivre faite en référence aux dessins schématiques annexés donnés uniquement à titre d'exemple illustrant un mode de réalisation de l'invention et dans lesquels :

La figure 1 est une vue simplifiée, schématique, en perspective, d'un appareil de radiodiagnostic selon la présente invention.

La figure 2 est une vue à plus grande échelle, avec arrachement, du détail indiqué en II sur la figure 1.

La figure 3 illustre les signaux de sortie du détecteur de vitesse des moyens de commande de décalage selon la présente invention.

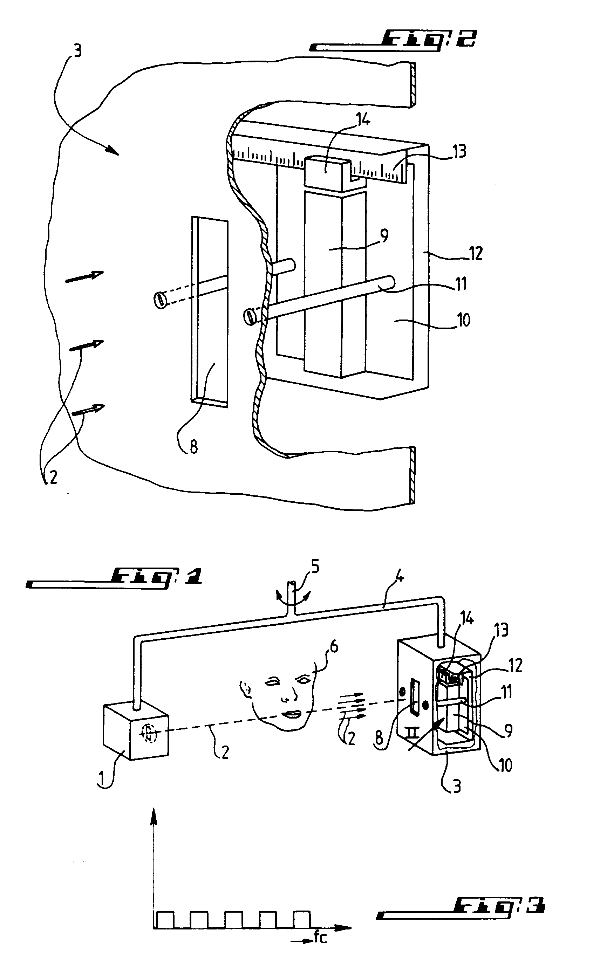

L'invention sera décrite, à titre d'exemple non limitatif, dans son application à un appareil de radiodiagnostic dentaire destiné à produire des prises de vue panoramiques tomographiques de la mâchoire d'un patient. Comme il ressort de la figure 1, un tel appareil comprend essentiellement un dispositif de source 1 des rayons X 2, un récepteur 3 des rayons X ainsi qu'un bras de support 4 du dispositif de source et du récepteur, qui est monté rotatif autour d'un axe 5 vertical disposé de façon à ce que l'appareil puisse tourner autour de la tête du patient 6 dont la mâchoire est traversée par les rayons X.

Le dispositif émetteur 1 de rayons X est du type connu en soi et n'a pas besoin d'être décrit plus en détail.

Le récepteur 3 comprend, comme cela est connu, une fente secondaire 8 à travers laquelle les rayons X 2 qui ont traversé la mâchoire et contiennent ainsi l'information des prises de vue panoramiques entrent dans le récepteur, pour parvenir sur un capteur 9 réalisé sous forme d'un dispositif à transfert de charge, par l'intermédiaire d'un écran scintillateur connu en soi et non représenté. Le capteur 9 est solidaire de la partie fixe 10 dont le déport mécanique par rapport à la fente est assuré par des moyens mécaniques 11.

Le capteur 9 du type à transfert de charge appelé CCD est également connu en soi et ne nécessite pas une explication détaillée. Il suffit de rappeler qu'il est du type matriciel, sensible aux rayons X et en mesure d'intégrer l'information constituée par les atténuations des rayons X ayant traversé la mâchoire, localement par accumulation de charges électriques dans les photosites du capteur qui constituent les éléments d'image et sont situés dans le prolongement du point d'impact photo-électrique. La lecture du capteur s'effectue par transfert des charges d'une ligne d'éléments d'image dans la ligne voisine, et ceci jusqu'au bord de la zone d'image. La dernière ligne d'éléments d'image est transférée dans un registre à décalage, qui est ensuite vidée élément par élément dans un amplificateur de sortie. Chaque élément d'image est ensuite converti après amplification, puis mémorisé en vue de sa présentation ou de son traitement numérique. Ce traitement ne fait pas partie de la présente invention, il est connu et ne sera donc pas décrit.

La particularité de l'invention réside dans le fait que le récepteur comporte une partie mobile 12 qui est adaptée pour être entraînée à la vitesse d'un film radiographique classique. A cette fin, cette partie mobile peut être translatée dans le porte cassette d'un appareil classique à film radiographique exactement comme une cassette, en coulissant sur la partie fixe 10. La partie mobile peut présenter les dimensions hors-tout d'une cassette porte-film standard.

Pour déterminer la vitesse de la partie mobile 12, celle-ci est pourvue d'une règle graduée 13 disposée de façon à passer devant un détecteur 14 du défilement desdites graduations, solidaire de la partie fixe. Le détecteur 14 est réalisé sous forme d'un codeur incrémental.

On comprend aisément que le détecteur codeur incrémental 14 produit un signal en forme d'une suite d'impulsions représentées à la figure 3, lorsque la règle graduée 13 défile devant lui lors d'un déplacement de la partie mobile 12. La fréquence $f_c$ du signal produit par le détecteur codeur incrémental 14 dépend de la résolution spatiale de celui-ci. La fréquence de commande du décalage des lignes du capteur CCD par rapport à la fréquence $f_c$ est déterminée par les dimensions des éléments d'image du capteur et peut être exprimée par la relation :

$$f_d = r/l.f_c$$

r étant essentiellement le pas de graducation de la règle et l la largeur d'un élément d'image.

Le signal de commande de décalage, ayant la fréquence de décalage $f_d$ est engendré par un dispositif approprié (non représenté) relié au détecteur 14. Ainsi à chaque fois que la partie mobile 12 s'est déplacée sur une longueur équivalente à la largeur d'un élément d'image, les lignes du capteur sont décalées d'une position.

Dans l'exemple représenté, le détecteur de vitesse 14 est réalisé sous forme d'un codeur incrémental optique. Bien entendu le détecteur peut être réalisé sous toute autre forme appropriée, par exemple sous forme d'un codeur à effet Hall.

Le détecteur 14 peut être adapté pour mesurer des déplacements angulaires de la partie mobile 12 et permet ainsi l'application de l'invention à des appareils orthopantomographes à tambour rotatif.

Il ressort de la description qui vient d'être faite, que l'invention peut être utilisée dans des appareils orthopantomographes du marché conçus pour utiliser des films radiographiques à déplacement linéaire ou rotatif, tout en utilisant les avantages des capteurs du type à transfert de charge CCD commandés pour fonctionner en mode d'intrégration à retard, appelés communément TDI.

Un appareil classique est aisément transformable en un appareil selon l'invention. Il convient notamment d'ajouter le capteur DTC 9 et ses organes de gestion du signal, les dispositifs de détection 14 et de conversion, et de prévoir sur la cassette la règle graduée 13.

## Revendications

1. Appareil de radiodiagnostic pour établir des prises de vue panoramiques et avantageusement tomographiques d'un objet (6) tel que de la mâchoire d'un patient, cet appareil comprenant une source (1) de rayons X(2) et un récepteur (3) des rayons X ayant traversés ledit objet, le récepteur comprenant une fente secondaire (8) de passage des rayons X reçus et, en aval d'un écran scintillateur transformant ces rayons X reçus en lumière visible, un capteur (9) du type DTC prévu pour être lu par transfert des charges électriques accumulées dans les éléments d'image d'une ligne dans la ligne suivante, qui est monté fixe dans le récepteur de façon que sa zone d'image corresponde à l'ouverture de la fente secondaire, des moyens de commande du transfert des charges par décalage des lignes de charge, à une fréquence simulant le déplacement d'un film radiographique classique derrière la fente secondaire, un dispositif de traitement des données fournies par le capteur et un dispositif de reproduction d'images, **caractérisé en ce que** les moyens de commande du décalage comprennent une partie mobile (12) se déplaçant derrière la fente secondaire (8) à la vitesse à laquelle se déplacerait un film radiographique et un détecteur (14) de la vitesse de déplacement de cette partie mobile (12) ainsi qu'une unité de conversion de la vitesse ainsi établie en une fréquence ($f_d$) équivalente de décalage des lignes du capteur à transfert de charges (9).

2. Appareil de radiodiagnostic selon la revendication 1, **caractérisé en ce que** la partie mobile (12) comprend un élément tel qu'une règle graduée (13) disposée de façon à défiler devant le détecteur (14) lors du déplacement de la partie mobile (12) et le détecteur (14) est adapté pour produire un signal dont la fréquence ($f_c$) est fonction de la graduation de l'élément (13), qui est transmis à l'unité de conversion précitée.

3. Appareil de radiodiagnostic selon la revendication 2, **caractérisé en ce que** le détecteur (14) est réalisé sous forme d'un codeur incrémental optique.

**4.** Appareil de radiodiagnostic selon la revendication 2, **caractérisé en ce que** le détecteur (14) est du type codeur à effet Hall.

**5.** Appareil de radiodiagnostic selon l'une des revendications 2 à 4, **caractérisé en ce que** l'unité de conversion est adaptée pour produire à partir du signal à la fréquence ($f_c$) produite par le détecteur (14), le signal de décalage en prenant en compte le pas du détecteur codeur (14) et la largeur des éléments d'image du capteur (9).

**6.** Appareil de radiodiagnostic selon la revendication 5, **caractérisé en ce que** la fréquence ($f_d$) du signal de décalage et la fréquence ($f_c$) du signal produit par le détecteur (14) répondent à la relation $f_d = r/l.f_c$, où r et l sont respectivement le pas de la graduation de la règle (13) et la largeur d'un élément d'image.


## Claims

**1.** Radiodiagnostic device to take panoramic and advantageously tomographic views of an object (6), such as the jaw of a patient, this device including a source (1) of X-rays (2) and an X-ray receiver (3) having traversed said object, the receiver including a secondary aperture 8 for passage of the X-rays received and, downstream of a scintillator screen, transforming these X-rays received into a visible light, a sensor (9) of the DTC type being provided to be read via the transfer of electric charges accumulated in the image elements of a line in the next line, said sensor being mounted fixed in the receiver so that its image zone corresponds to the opening of the secondary aperture, means for controlling the transfer of the charges by shifting the charge lines on a frequency simulating the movement of a conventional radiographic film behind the secondary aperture, a device for processing the data provided by the sensor and an image reproduction device, **characterised in that** the shift control means include a mobile portion (12) moving behind the secondary aperture (8) at a speed at which a radiographic film would move, and a detector (14) for detecting the speed of movement of this mobile portion (12), as well as a unit for converting the speed established into an equivalent frequency ($f_d$) for shifting the lines of the charge transfer sensor (9).

**2.** Radiodiagnostic device according to claim 1, **characterised in that** the mobile portion (12) includes an element, such as a line rule (13) placed in such a way to run off in front of the detector (14) during movement of the mobile portion (12), the detector (14) being adapted to produce a signal whose frequency ($f_c$) depends on the graduation of the element (13) which is transmitted to said conversion unit.

**3.** Radiodiagnostic device according to claim 2, **characterised in that** the detector (14) is embodied in the form of an optical incremental encoder.

**4.** Radiodiagnostic device according to claim 2, **characterised in that** the detector (14) is a Hall effect encoder type detector.

**5.** Radiodiagnostic device according to one of claims 2 to 4, **characterised in that** the conversion unit is adapted to produce on the basis of the signal at the frequency ($f_c$) produced by the detector (14) the shift signal by taking into account the step of the encoder detector (14) and the width the image elements of the sensor (9).

**6.** Radiodiagnostic device according to claim 5, **characterised in that** the frequency ($f_d$) of the shift signal and the frequency ($f_c$) of the signal produced by the detector (14) satisfy the equation $f_d = r/l.f_c$ where r and 1 are respectively the step of the graduation of the rule (13) and the width of an image element.


## Patentansprüche

**1.** Röntgendiagnostikeinrichtung zum Erstellen von Panoramaaufnahmen und vorteilhaft tomographischen Aufnahmen eines Objekts (69) wie zum Beispiel des Kiefers eines Patienten, wobei diese Einrichtung eine Quelle (1) für Röntgenstrahlen (2) und ein Aufnahmegerät (3) für Röntgenstrahlen umfaßt, die dieses Objekt durchdringen haben, wobei das Aufnahmegerät einen sekundären Durchgangsschlitz (8) der empfangenen Röntgenstrahlen umfaßt, und unterstromig einen Szintillatorbildschirm, der diese empfangenen Röntgenstrahlen in sichtbares Licht umwandelt, einen Sensor (5) von der Art DTC, der vorgesehen ist, um per Transfer der elektrischen Ladungen gelesen zu werden, die sich in den Bildelementen einer Linie in der nächsten Linie angesammelt haben, der fest im Aufnahmegerät derart angebracht ist, daß sein Bildbereich der Öffnung des sekundären Schlitzes entspricht, Steuermittel für den Transfer der Ladungen durch Versetzen der Ladelinien bei einer Frequenz, die die Verschiebung eines klassischen Röntgenfilms hinter dem sekundären Schlitz simuliert, eine Vorrichtung zur Verarbeitung der Daten, die vom Sensor und einer Bildwiedergabevorrichtung geliefert werden, **dadurch gekennzeichnet, daß** die Steuermittel der Versetzung einen mobilen Teil (12) umfassen, der hinter dem sekundären Schlitz (8) in der Geschwindigkeit verschoben wird, in der sich ein Röntgenfilm und ein Detektor (14) für die Verschiebegeschwindigkeit dieses mobilen Teils (12) verschieben würde, sowie eine Umwandlungseinheit der Geschwindigkeit, die somit in einer Frequenz ($f_d$) festgelegt wird, die der Verschiebung der Linien des Sensors für den Ladungstransfer (9) entspricht.

2. Radiodiagnostikeinrichtung gemäß Anspruch 1, **dadurch gekennzeichnet, daß** der mobile Teil (12) ein Element, wie zum Beispiel ein skaliertes Linea (13) umfaßt, das derart angeordnet ist, daß es vor dem Detektor (14) bei der Verschiebung des mobilen Teils (12) abläuft, und der Detektor (14) ist angepaßt, um ein Signal zu produzieren, dessen Frequenz ($f_c$) von der Skalierung des Elements (13) abhängt, das an die vorgenannte Umwandlungseinheit übertragen wird.

3. Radiodiagnostikeinrichtung gemäß Anspruch 2, **dadurch gekennzeichnet, daß** der Detektor (14) in Form eines optischen Inkrementcodierers realisiert ist.

4. Radiodiagnostikeinrichtung gemäß Anspruch 2, **dadurch gekennzeichnet, daß** der Detektor (14) vom Typ Codierer mit Halleffekt ist.

5. Radiodiagnostikeinrichtung gemäß Anspruch 2 bis 4, **dadurch gekennzeichnet, daß** die Umwandlungseinheit angepaßt ist, um , ausgehend von dem Signal mit der vom Detektor (14) produzierten Frequenz ($f_c$), das Versetzungssignal zu produzieren und dabei den Schritt des Codierdetektors (14) und die Breite der Bildelemente des Sensors (9) zu berücksichtigen.

6. Radiodiagnostikeinrichtung gemäß Anspruch 5, **dadurch gekennzeichnet, daß** die Frequenz ($f_d$) des Versetzungssignals und die Frequenz ($f_c$) des Signals, das vom Detektor (14) produziert wird, dem Verhältnis $f_d = r/l.f_c$ entsprechen, wobei r und l jeweils der Skalierungsschritt des Lineals (13) und die Breite eines Bildelements sind.

**Fig. 2**

**Fig. 1**

**Fig. 3**